(19) **Europäisches Patentamt**
European Patent Office
Office européen des brevets

(11) **EP 2 552 404 B1**

(12) **EUROPÄISCHE PATENTSCHRIFT**

(45) Veröffentlichungstag und Bekanntmachung des Hinweises auf die Patenterteilung:
**21.01.2015 Patentblatt 2015/04**

(21) Anmeldenummer: **11714245.5**

(22) Anmeldetag: **28.03.2011**

(51) Int Cl.:
*A61K 31/567* (2006.01)      *A61K 31/4196* (2006.01)
*A61K 31/00* (2006.01)      *A61K 31/569* (2006.01)
*A61K 45/06* (2006.01)      *A61K 9/00* (2006.01)
*A61P 15/18* (2006.01)      *A61P 15/00* (2006.01)

(86) Internationale Anmeldenummer:
**PCT/EP2011/054737**

(87) Internationale Veröffentlichungsnummer:
**WO 2011/120925 (06.10.2011 Gazette 2011/40)**

(54) **PARENTERALE ARZNEIFORM, DIE AROMATASEHEMMER UND GESTAGENE FREISETZT, FÜR DIE BEHANDLUNG VON ENDOMETRIOSE**

PARENTERAL PHARMACEUTICAL FORM WHICH RELEASES AROMATASE INHIBITOR AND GESTAGENS, FOR THE TREATMENT OF ENDOMETRIOSIS

FORME GALÉNIQUE PARENTÉRALE LIBÉRANT DES INHIBITEURS DE L'AROMATASE ET DES GESTAGÈNES, POUR LE TRAITEMENT DE L'ENDOMÉTRIOSE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**

(30) Priorität: **31.03.2010 DE 102010003494**

(43) Veröffentlichungstag der Anmeldung:
**06.02.2013 Patentblatt 2013/06**

(73) Patentinhaber: **Bayer Intellectual Property GmbH**
**40789 Monheim (DE)**

(72) Erfinder:
• **PAKKALIN, Arto**
**00170 Helsinki (FI)**
• **KNAUTHE, Rudolf**
**16548 Glienicke (DE)**
• **SCHMITZ, Heinz**
**10715 Berlin (DE)**
• **TALLING, Christine**
**FI-20610 Turku (FI)**
• **JUKARAINEN, Harri**
**FI-21620 Kuusisto (FI)**
• **KOROLAINEN, Henriikka**
**FI-32210 Loimaa (FI)**

(74) Vertreter: **BIP Patents**
**c/o Bayer Intellectual Property GmbH**
**Creative Campus Monheim**
**Alfred-Nobel-Straße 10**
**40789 Monheim (DE)**

(56) Entgegenhaltungen:
**WO-A1-2004/069260      WO-A2-02/072106**
**US-A1- 2005 101 579      US-A1- 2011 033 519**

• **AMSTERDAM ET AL: "Anastrazole and oral contraceptives: a novel treatment for endometriosis", FERTILITY AND STERILITY, ELSEVIER SCIENCE INC, NEW YORK, NY, USA, Bd. 84, Nr. 2, 1. August 2005 (2005-08-01) , Seiten 300-304, XP005061109, ISSN: 0015-0282, DOI: DOI:10.1016/J.FERTNSTERT.2005.02.018 in der Anmeldung erwähnt**
• **REMORGIDA ET AL: "Letrozole and norethisterone acetate in rectovaginal endometriosis", FERTILITY AND STERILITY, ELSEVIER SCIENCE INC, NEW YORK, NY, USA, Bd. 88, Nr. 3, 1. September 2007 (2007-09-01), Seiten 724-726, XP022238505, ISSN: 0015-0282, DOI: DOI:10.1016/J.FERTNSTERT.2006.12.027**

- SHIPPEN EUGENE R ET AL: "Successful treatment of severe endometriosis in two premenopausal women with an aromatase inhibitor", FERTILITY AND STERILITY, Bd. 81, Nr. 5, Mai 2004 (2004-05), Seiten 1395-1398, XP002638841, ISSN: 0015-0282
- LANDGREN B M ET AL: "Pharmacokinetic and pharmacodynamic effects of vaginal rings releasing levonorgestrel at a rate of 27 mug/24 hours: A pilot study", CONTRACEPTION, GERON-X, INC., LOS ALTOS, CA, US, vol. 49, no. 2, 1 February 1994 (1994-02-01), pages 139-150, XP026208597, ISSN: 0010-7824, DOI: 10.1016/0010-7824(94)90089-2 [retrieved on 1994-02-01]
- VERCELLINI P ET AL: "Comparison of contraceptive ring and patch for the treatment of symptomatic endometriosis", FERTILITY AND STERILITY, ELSEVIER SCIENCE INC, NEW YORK, NY, USA, vol. 93, no. 7, 1 May 2010 (2010-05-01), pages 2150-2161, XP027021065, ISSN: 0015-0282 [retrieved on 2009-03-27]
- HEFLER L A ET AL: "Role of the vaginally administered aromatase inhibitor anastrozole in women with rectovaginal endometriosis: a pilot study", FERTILITY AND STERILITY, ELSEVIER SCIENCE INC, NEW YORK, NY, USA, vol. 84, no. 4, 1 October 2005 (2005-10-01), pages 1033-1036, XP027699728, ISSN: 0015-0282 [retrieved on 2005-10-01]
- "Pharmacokinetics of anastrozole and tamoxifen alone, and in combination, during adjuvant endocrine therapy for early breast cancer in postmenopausal women: A sub-protocol of the 'ArimidexTM and Tamoxifen Alone or in Combination' (ATAC) trial", BRITISH JOURNAL OF CANCER, NATURE PUBLISHING GROUP, GB, vol. 85, no. 3, 1 January 2001 (2001-01-01), pages 317-324, XP009005805, ISSN: 0007-0920, DOI: 10.1054/BJOC.2001.1925

## Beschreibung

Inhalt der vorliegenden Erfindung für die Behandlung von Endometriose ist die Bereitstellung einer parenteralen Arzneiform (Abgabesystem) für die kontrollierte Freisetzung von Anastrozol mit einer Rate, die keine Stimulation der Ovarien durch negative Rückkopplung der Hypophysen-Ovar-Achse induziert (keine Steigerung der Sekretion von Gonadotropinen, die eine Stimulation von Follikelwachstum induzieren würde), und Levonorgestrel mit einer Rate, die eine empfängnisverhütende Wirkung auf der Grundlage bekannter lokaler Wirkungen bereitstellt (wie z. B. Verringern und Eindicken des Zervixschleims, um das Aufsteigen von Spermien zu behindern, Wirkungen auf das Endometrium und die Tubenmotilität, um die Implantation und den Transport von Eizellen zu behindern). Die Kombination eines AI und eines Gestagens in ovulationshemmender Dosierung würde aufgrund starker Unterdrückung der körpereigenen Östrogensynthese zu Östrogenmangelsymptomen führen (z. B. Hitzewallungen, Abnahme der Knochendichte). Aufgrund der in dieser Erfindung verwendeten niedrigen Dosierungen (Anastrozol ohne Gegenregulation und Levonorgestrel ohne zuverlässige Hemmung der Ovulation), wird das Risiko von Östrogenmangelsymptomen durch die Kombination wirksam minimiert. Die Arzneiform, die hier beschrieben wird, ist eine Arzneiform auf Polymerbasis, die wenigstens eine Kammer umfasst, wobei die eine Kammer oder alle Kammern einen Kern oder einen von einer Membran umhüllten Kern umfasst/umfassen, wobei der Kern und die Membran im Wesentlichen aus der gleichen oder aus unterschiedlichen Polymerzusammensetzungen bestehen, wobei wenigstens eine Kammer Anastrozol umfasst und wenigstens eine Kammer, die die gleiche Kammer sein kann, die ds Anastrozol umfasst, oder davon verschieden, ein Levonorgestrel umfasst. Die parenterale Arzneiform, die für die Abgabe von therapeutischen Wirkstoffen mit einer kontrollierten Freisetzungsrate über einen ausgedehnten Zeitraum geeignet ist, ist einintravaginalring [IVR; die Begriffe Intravaginalring und Vaginalring werden synonym verwendet]) wobei die Anwendungsdauer 1 Woche bis 3 Monate, vorzugsweise 4 bis 6 Wochen.beträgt. Ein IVR, bietet den zusätzlichen Vorteil, zusätzliche lokale Wirkungen an endometriotischen Läsionen in der Nähe der Applikationsstelle zu entfalten.

Endometriose ist eine chronische Erkrankung, die etwa 10 % der Frauen im gebärfähigen Alter betrifft. Die Erkrankung ist durch das Vorhandensein von endometriumartigem Gewebe außerhalb der Uterushöhle gekennzeichnet. Es gibt verschiedene Theorien über die Pathogenese der Endometriose. Wahrscheinlich wird sie in den meisten Fällen durch eine retrograde Menstruation, bei der endometriales Gewebe durch die Eileiter in die Bauchhöle gelangt, ausgelöst, wo die endometrialen Zellen an die Oberfläche von abdominalen Geweben und Organen anhaften, um ektopische endometriale Implantate zu bilden, d. h. endometriotische Läsionen.

Dieses endometriumartige Gewebe kann auf die gleiche Weise wie normales Endometrium auf Veränderungen der hormonellen Umgebung während des Menstruationszyklus antworten, so dass sich das Gewebe auf die gleiche Weise wie das Endometrium selbst bei den Veränderungen der Östrogen- und Progesteronkonzentrationen verhält. Im Verlauf der Erkrankung können sich diese endometriotischen Läsionen aber vom normalen Menstruationszyklus abkoppeln. Das Vorhandensein von endometrialen Implantaten auf abdominalen Oberflächen (Endometrioseherde) kann entzündliche Reaktionen auslösen, die zusammen mit dem Einwachsen von Nervenfasern die pathophysiologischen/anatomischen Entsprechungen für die typischen, mit Endometriose verbundenen Symptome, wie z. B. Unterleibsschmerz, Dysmenorrhö und Dyspareunie, darstellen können.

Aktuelle, bei Endometriose verwendete Behandlungen beruhen auf der Hemmung der ovariellen Östrogenproduktion durch zentrale Hemmung der Hypophysen-Ovar-Achse (beispielsweise Analoga des Gonadotropin-freisetzenden Hormons [GnRH-Analoga], Danazol, Medroxyprogesteronacetat, Dienogest, kombinierte orale Kontrazeptiva (COCs "combined oral contraceptives"). Die Hemmung der ovariellen Östrogenproduktion führt jedoch bei Behandlung mit GnRH-Analoga zu Nebenwirkungen, die mit Östrogenmangel in Verbindung stehen, wie z. B. Hitzewallungen und Knochenabbau als die wichtigsten, wenn bei der Behandlung kein Östrogen zugegeben wird. Andere Nebenwirkungen können umfassen: vorübergehende Vaginalblutung, Vaginaltrockenheit, verminderte Libido, Brustspannen, Schlaflosigkeit, Depression, Reizbarkeit und Müdigkeit, Kopfschmerz und verminderte Elastizität der Haut. Zum Vermindern dieser Nebenwirkungen unter GnRH-Analogatherapie wurden sogenannte Add-back-Behandlungspläne entwickelt, bei denen zu der Therapie mit GnRH-Analoga (konjugierte) Östrogene oder Norethisteronacetat (NETA, das teilweise zu Östradiol metabolisiert wird) zugegeben werden. Beide Therapien (GnRH-Analoga + Östrogen oder GnRH-Analoga + NETA) werden mit ihrer vollen wirksamen Dosis angewendet, was auch bedeutet, dass das gesamte Spektrum von zu erwartenden Nebenwirkungen dieser Medikationen auftreten kann. COCs allein gegeben sind auch bei der Behandlung von Endometriose wirksam und erfordern keine Add-Back-Therapie.

Wie bei Add-back-Behandlungsplänen auch, wird durch die Behandlung mit COCs der Patientin exogenes Östrogen zugeführt, hier das starke Östrogen Ethinylestradiol. Die exogene Östrogenzufuhr kann dabei theoretisch die Wirksamkeit des Gestagens oder des GnRH-Analogon gegen die östrogenabhängige Erkrankung Endometriose beeinträchtigen.

Andererseits hat das Hemmen der Hypophysen-Ovar-Achse keinen Einfluss auf Orte der Östrogenproduktion außerhalb der Ovarien, was für neue Behandlungsmodalitäten der Endometriose entscheidende Bedeutung haben könnte. Bei früheren Untersuchungen wurde gefunden, dass das Enzym Aromatase, das die Umwandlung von Testosteron und anderen androgenen Vorläufern zu Östrogen katalysiert, in endometriotischen Läsionen exprimiert wird (Urabe M et al., Acta Endocrinol. (Copenh.) 1989, 121(2): 259-64, Noble LS et al., J. Clin. Endocrinol. Metab. 1996, 81(1): 174-9). Folglich können endometriotische Läsionen lokal erhebliche Mengen an Östradiol produzieren, was Behandlungsversagen der oben genannten Therapien erklären könnte, die lediglich die ovarielle Östrogenproduktion hemmen. Zusätzlich wurde gezeigt, dass der Entzündungsmediator Prostaglandin E2 als wirkungsvoller Stimulator der Aromataseexpression wirkt und die lokale Östrogenproduktion in dem entzündlichen Milieu endometriotischer Läsionen weiter verstärkt (Noble LS et al., J. Clin. Endocrinol. Metab. 1997, 82(2): 600-6).

Bei typischen Dosierungen (beispielsweise 1 mg/Tag Anastrozol) verringern AIs die systemischen Östrogenspiegel von Frauen nach der Menopause mehr als > 85 % (Geisler J et al., J. Clin. Oncol. 2002, 20(3): 751-757). Bei Frauen vor der Menopause wird diese Wirkung durch Gegenregulierung über die Hypophysen-Ovar-Achse vermindert (d. h. die hypophysäre Wahrnehmung verminderter systemischer Östrogenspiegel führt zur Sekretion von Gonadotropinen, die die Östrogensynthese in den Ovarien stimulieren und die Wirkung des AI teilweise aufheben), wodurch das ovarielle Follikelwachstum angeregt wird (diese Wirkung wird bei Patientinnen genutzt, die an ovarieller Subfertilität leiden, um das Follikelwachstum anzuregen). Aus diesem Grund wurden bei mehreren klinischen Versuchen bei Patientinnen mit Endometriose AIs in Dosierungen verwendet, die typischerweise bei Frauen nach der Menopause zur Behandlung von Brustkrebs verwendet werden, in Kombination mit Arzneimitteln, die die Gegenregulierung hemmen, beispielsweise mit NETA (Ailawadi RK et al., Fertility & Sterility 2004, 81(2): 290-296) oder COCs (Amsterdam LL et al. 2005, Fertility & Sterility 2005, 84(2): 300-304). Bei diesen Kombinationen wird neben der Hemmung der Gegenregulierung die Verringerung der mit Östrogenmangel verbundenen Nebenwirkungen als Vorteil angesehen. Die Verabreichung von exogenem Östrogen oder NETA in diesen Kombinationen kann aber die Wirksamkeit (siehe oben) von AIs bei der Behandlung der Symptome der Endometriose verringern.

WO 03/15872 beschreibt ein Verfahren zum Behandeln oder Verhindern von uterinen Fibroiden oder Endometriose durch intravaginale Verabreichung eines AI an eine Patientin. Die Erfindung offenbart den Vorteil lokaler Wirkungen einer Monotherapie mit AIs und beansprucht die Verringerung systemischer Nebenwirkungen durch die lokale Verabreichung. Die Anmeldung offenbart nicht die Kombination eines AIs mit einem Gestagen in der Form einer parenteralen Arzneiform, insbesondere nicht eine Kombination eines AIs mit einem Gestagen in einem IVR oder IUD. Im Gegensatz zu der vorliegenden Erfindung offenbart WO 03/15872 kein Mittel zum Erzielen einer empfängnisverhütenden Wirkung, die bei der vorliegenden Erfindung entscheidend ist, da es bei einem sinnvollen Produktprofil von entscheidender Bedeutung ist, eine Schwangerschaft zu verhindern, wenn eine Frau im gebärfähigen Alter unter Behandlung mit einem AI steht. Die bei der vorliegenden Erfindung beschriebene technische Lösung ist das Kombinieren des AIs und der empfängnisverhütenden Wirkung eines Gestagens in einer parenteralen Arzneiform, um das physikalische Trennen der beiden zu verhindern und so die Möglichkeit auszuschließen, dass ein AI ohne empfängnisverhütenden Schutz für die Behandlung von Endometriose verwendet wird. Diese Möglichkeit wird nicht ausgeschlossen, wenn zwei physikalisch trennbare Arzneiformen verwendet werden.

Auch die Kombination eines AIs mit einem Gestagen (AI + NETA, Ailawadi RK et al., 2004) oder mit einem COC (Amsterdam LL et al., 2005; WO 04/69260) wurde für die orale Verwendung vorgeschlagen. Beide Kombinationen sollen Symptome des Östrogenmangels durch exogene Verabreichung von Östrogenaktivität verhindern (Östrogenmetabolismus von NETA; Ethinylestradiol in COCs). Der Nachteil dieser Behandlungsmodalitäten und der Differenzierung der in dieser Anmeldung beschriebenen Erfindung liegt darin, dass in beiden Fällen die Verabreichung von exogener Östrogenaktivität (NETA wird teilweise zu Östrogenen umgewandelt; COCs enthalten das starke Östrogen Ethinylestradiol) notwendig ist, um Nebenwirkungen zu verhindern. Dadurch wird die pharmakodynamische Wirkung des AIs auf endometriotisches Gewebe abgeschwächt. Ferner beschreiben diese Offenbarungen nicht die Vorteile einer lokalen Anwendung des AIs, die die lokal exprimierte Aromatase von endometrialen Läsionen in der Umgebung der Arzneiform hemmt, wodurch die zum Erzielen der gewünschten vollen pharmakologischen Wirkung notwendige Dosis verringert wird.

L.A. Hefler et. al. haben in einer Pilotstudie die Rolle des Anastrozols in Patientinnen mit rectovaginaler Endometriose untersucht [Fertility and sterility (ISSN: 0015-0282) Band: 84 (2005)]. Auch Hefler kommt ohne die Zugabe von exogenem Östrogen bei der Behandlung der Endometriose aus. Hefler offenbart jedoch keine hormonhaltigen Kontrazeptiva zu verwenden und hat nicht erkannt, dass Gestagene eingesetzt werden können, wenn diese wie im Falle der vorliegenden Erfindung niedrig dosiert und gemeinsam mit Anastrozol lokal verabreicht werden. Darüber hinaus verabreicht Heller in der beschriebenen Studie zusätzlich noch 1,2 Gramm Kalzium sowie 800 IE Vitamin D (Cholecalciferol), um eine ausreichende vaginale Aufnahme des Anastrozols sicherzustellen.

US 2005/0101579 (Shippen et al.) offenbart Vaginal oder Rektalsuppositorien zur Endometriosebehandlung, die ein Aromatasehemmer und das Gestagen Progesteron enthalten. In der offenbarten Fallstudie werden 1 mg Anastrozol und 200 mg Progesteron und zusätzlich auch 0,5 μg Calcitriol und 12,5 mg Rofecoxib täglich oral verabreicht. Das dort beschriebene Regime führte jedoch zu einer Vaginalatrophy aufgrund Estrogenmangels, so dass nach den ersten sechs Behandlungswochen eine Estrogengabe erforderlich wurde. Ein solches "add back" Regime wird im Falle der vorliegenden Erfindung verhindert.

Der Erfindung, die in der vorliegenden Anmeldung beschrieben wird, kommt möglicherweise die Patentanmeldung WO 03/17973 am nächsten, die die Anwendung von AIs auf dem vaginalen Weg offenbart, allein oder in Kombination mit anderen Verbindungen, die den Östrogenmetabolismus beeinflussen, wie z. B. Cyclooxygenase-2-Hemmer (COX-2-Hemmer) und 17-beta-Hydroxysteroiddehydrogenase-1-Hemmer (17ßHSD-1-Hemmer). Ferner beansprucht die Erfindung ein Verfahren, bei dem die ovarielle Östrogensynthese nicht gehemmt wird. Die Erfindung offenbart den Vorteil von Kombinationen von AIs mit anderen Arzneimitteln, die dem Östrogenmetabolismus beeinflussen, über lokale Applikation. Die Anmeldung offenbart nicht die Kombination eines AIs mit einem Gestagen in der Form einer parenteralen Arzneiform, insbesondere nicht eine Kombination eines AIs mit einem Gestagen in den beschriebenen Dosierungen in einem IVR. Im Gegensatz zu der vorliegenden Erfindung offenbart WO 03/17973 kein Mittel zum Erzielen einer empfängnisverhütenden Wirkung. Auch hier ist es wichtig, zu erkennen, dass nur die physikalisch untrennbare Kombination der AI-Wirksamkeit mit der empfängnisverhütenden Wirkung zu einem sinnvollen Produkt führt.

In US 2011/0033519 A1 (Publikationsdatum: 10. Februar 2011) werden Arzneiformen beschrieben, die Aromataseinhibitoren ggf. in Kombination mit kontrazeptiv wirkenden Substanzen lokal ins Uterusgewebe abgeben. Damit sollen Krankheiten wie Myome, Adenomyose und Endometriose behandelt oder vorgebeugt werden. Da Gestagene das Wachstum von Myomen stimulieren könnten, wird von deren Verwendung abgeraten und statt dessen Kupfer und andere Edelmetalle als kontrazeptives Prinzip bevorzugt. Geeignete IUD-Aromataseinhibitordosen werden - z.B. für Anastrozol - mit 1 µg - 10 mg/Tag angegeben. Bei einer dort vorgeschlagenen Verwendungsdauer von 5-10 Jahren scheint dies jedoch technisch kaum umsetzbar.

Ein Aspekt der in der vorliegenden Anmeldung beschriebenen Erfindung bei Verwendung eines IVR beruht auf dem Konzept der lokalen Anwendung einer Dosis eines AIs, die keine gegenregulierenden Wirkungen der Hypophysen-Ovar-Achse induziert, aber ihre aromatasehemmende Wirkung in den endometriotischen Läsionen zeigt. Ohne gegenregulierende Wirkungen als Folge der Anastrozol-Verabreichung besteht keine Notwendigkeit zur Anwendung eines Gestagens oder COCs zum Hemmen der Hypophysen-Ovar-Achse, wodurch eine Dosisverringerung des Gestagens auf die Dosis möglich wird, die zum Erzielen einer empfängnisverhütenden Wirkung durch lokale Mechanismen notwendig ist. Auf diese Weise werden Symptome des Östrogenmangels vermieden und es wird keine Verabreichung von exogenem Östrogen notwendig sein. Da Endometriose eine östrogenabhängige Erkrankung ist, wird durch die fehlende Verabreichung von exogenen Östrogenen die therapeutische Wirksamkeit des Anastrozols nicht beeinträchtigt. Da auch Gestagene eine Hemmwirkung auf die Aromataseexpression haben, könnte das Gestagen in der vorliegenden Erfindung zur Wirkung des Anastrozols beitragen.

Um einerseits gegenregulierende Wirkungen der Hypophysen-Ovar-Achse bei der größtmöglichen Dosis des Anastrozols zu vermeiden und andererseits die beste empfängnisverhütende Wirksamkeit der nur auf Gestagen beruhenden Empfängnisverhütung mit der größtmöglichen Gestagendosis unterhalb der ovulationshemmenden Dosis zu erzielen, müssen die Wirkstoffe in einer Formulierung mit kontrollierter Freisetzung angewendet werden, die große Fluktuationen der Serumspiegel vermeidet, die eine Gegenregulierung durch die Hypophysen-Ovar-Achse auslösen könnten. Dies wird durch eine parenterale Arzneiform erreicht, vorzugsweise durch einen IVR.

Auf diese Weise wird bei der Erfindung, die in der vorliegenden Anmeldung beschrieben wird, eine wirkungsvolle Behandlung von Endometriose mit einem zuverlässigen empfängnisverhütenden Verfahren in einem Anwendungsmodus kombiniert, der durch eine parenterale Arzneiform zu einer hohen Compliance beiträgt (keine Anastrozol-Aufnahme ohne empfängnisverhütenden Schutz und daher keine unbeabsichtigte Exposition eines Embryos gegenüber Anastrozol). Im Gegensatz zu den im Stand der Technik beschriebenen Verfahren wird die Kombination gemäß der vorliegenden Erfindung die Arzneimittelexposition sowohl gegenüber dem anastrozol als auch gegenüber Levonorgestrel auf die Menge verringert, die für die Wirksamkeit notwendig ist, wodurch auch das Risiko ungünstiger Nebenwirkungen, die mit herabgesetzten Östrogenspiegeln verbunden sind, beispielsweise Hitzewallungen, Knochenabbau usw., minimiert wird.

Um das Risiko von Nebenwirkungen, die mit Östrogenmangel verbunden sind, zu minimieren, wird die bei der vorliegenden Erfindung angestrebte Levonorgestrelexposition unterhalb der Exposition liegen, die durch Verabreichung von Levonorgestrel mit ovulationshemmender Dosis erzielt wird (unabhängig vom Verabreichungsweg), aber hoch genug, um empfängnisverhütende Wirksamkeit durch lokale Wirkungen bereitzustellen, wie beispielsweise anhand des Insler-Scores gemessen wird (Insler V et al., Int. J. Gynecol. Obstet. 1972, 10: 223-228). Die oralen ovulationshemmenden Dosen verschiedener Gestagene und Levonorgestrel- die nach oraler Verabreichung zu bestimmten, gestagenspezifischen Plasma- bzw. Serumkonzentrationen führen - werden in der Literatur beschrieben, wie z. B. in Neumann F et al., Reproduktionsmedizin, 1998, 14: 257-264, und Taubert H D, Kuhl, H, Kontrazeption mit Hormonen, 2. Aufl., 1995. Zur Erläuterung: Die anastrozol-Dosis in der Kombination wird die Ovaraktivität nicht wesentlich über die typischen, nur auf Gestagen beruhenden Wirkungen stimulieren, die bei der vorliegenden Erfindung in der zu verabreichenden Gestagendosis erwartet werden. Der experimentelle Aufbau zum Bestimmen der Dosis von Levonorgestrel und Anastrozol wird im experimentellen Teil beschrieben.

Die Arzneiform gemäß der Erfindung, die eine Kombination von Anastrozol mit Levonorgestrel umfasst, ist besonders zur Behandlung von Endometriose geeignet, indem diese die Wirksamkeit gegen mit Endometriose verbundenen Symptomen bereitstellt und dabei das Risiko von mit Östrogenmangel verbundenen Nebenwirkungen (beispielsweise Knochenabbau, Hitzewallungen) minimiert. Zugleich stellt die Erfindung eine davon nicht physikalisch trennbare tägliche Exposition gegenüber Levonorgestrel bereit, um eine zuverlässige empfängnisverhütende Wirksamkeit zu gewährleisten und damit das Risiko einer Schwangerschaft mit nachfolgender unbeabsichtigter Exposition eines Embryos gegenüber anastrozol zu vermeiden. Dies ist ein Hauptgesichtspunkt der Erfindung, da es die Sicherheit des gewünschten Produkts in sinnvoller Weise verbessert (siehe im Gegensatz hierzu WO 03/15872 und WO 03/17973). Ferner kann bei der parenteralen/lokalen Anwendung in einer Arzneiform mit kontrollierter Freisetzungsrate, wie sie bei der Ausführungsform (IVR) verwirklicht wird, im Gegensatz zur oralen Anwendung eine geeignete Dosierung erzielt werden, um das gewünschte medizinische Ergebnis mit der bestmöglichen Verringerung der wichtigsten Nebenwirkungen, die mit einer fluktuierenden Exposition gegenüber den Wirkstoffen (Amplitude zwischen den größten Serumspiegeln, beispielsweise nach der Aufnahme oraler Formulierungen, und tiefen Serumspiegeln vor der nächsten Aufnahme) verbunden sind, zu erzielen. Außerdem kann die lokale Anwendung für die Behandlung von endometriotischen Läsionen in der Nähe der parenteralen Arzneiform besonders vorteilhaft sein (beispielsweise bei vaginaler Endometriose, tief infiltrierender Endometriose, Adenomyose oder Endometriose des cul-de-sac).

**Anastrozol** ist eine Verbindung, die die Wirkung des Enzyms Aromatase hemmt, das durch einen Aromatisierung genannten Vorgang Androgene in Östrogene umwandelt. Durch ihre Wirkung verringert oder blockiert Anastrozol die Synthese von Östrogenen.

Eine **parenterale Arzneiform** wie sie bei der Erfindung zur Anwendung kommt, ist ein IVR. Ein IVR ist eine im Wesentlichen ringförmige, polymere Arzneiform, das eine kontrollierte Freisetzung von Wirkstoffen an die Vagina über ausgedehnte Zeiträume bereitstellt.

**Freisetzungsrate** bedeutet die mittlere, während 24 Stunden aus der Arzneiform freigesetzte Menge an Wirkstoff, die dem umgebenden Gewebe zur Absorption zur Verfügung steht. Dem Fachmann ist bekannt, dass die mittlere Freisetzungsrate aus einer parenteralen Arzneiform über den Applikationszeitraum abnehmen kann.

**Arzneiform zur kontrollierten Langzeitfreisetzung** bedeutet eine beliebige Arzneiform, die für die Verabreichung von Arzneimitteln über einen ausgedehntem Zeitraum geeignet ist und die Fluktuationen von Arzneimittelspiegeln vermeidet, die normalerweise von Formulierungen mit sofortiger Freisetzung (beispielsweise Tabletten, Injektionen usw.) induziert werden.

Ein **Gestagen** wie es bei der vorliegenden Erfindung zur Anwendung kommt ist Levonorgestrel.

Ein **Gestagen in einer täglichen Freisetzungsrate, die unter der ovulationshemmenden Dosis liegt, aber hoch genug ist, um einen zuverlässigen empfängnisverhütenden Schutz bereitzustellen** bedeutet, dass bekannte Wirkungen, wie z. B. Verringerung und Eindickung des Zervixschleims, die das Aufsteigen von Spermien behindern, und Wirkungen auf das Endometrium und auf die Tubenmotilität, die die Implantation und den Transport von Eizellen behindern, die Befruchtung von Eizellen verhindern. Eine für diese Wirkung typische Gestagendosierung findet sich in dem Präparat Microlut® mit einer Tablettendosierung von 30 μg Levonorgestrel.

Typische orale ovulationshemmende Dosen sind (Neumann F et al., Reproduktionsmedizin, 1998, 14: 257-264; Taubert H D, Kühl, H, Kontrazeption mit Hormonen, 2. Aufl. 1995):

| Gestagen | ovulationshemmende Dosis [μg/Tag p.o.] | |
|---|---|---|
|  | Neumann et al. | Taubert &Kuhl |
| Norethisteron | 500 | 400 |
| Norethisteronacetat | 500 | |
| Lynestrenol | 2000 | 2000 |
| Norgestimat | 200 | 200 |
| Levonorgestrel | 50 | 60 |
| Desogestrel | 60 | 60 |
| Gestoden | 30 | 30 |
| Dienogest | 1000 | |
| Chlormadinonacetat | 1500-2000 | 1700 |
| Cyproteronacetat | 1000 | 1000 |
| Medroxyprogesteronacetat | 10 | |
| Drospirenon | 2000 | |
| 3-Keto-Desogestrel | | 60 |

Anmerkung: Dem Fachmann ist bekannt, dass die Werte der ovulationshemmenden Dosis von Gestagenen aus methodologischen und statistischen Gründen zu einem gewissen Maß variieren. Die bei der vorliegenden Erfindung verwendete Levonorgestreldosis/Exposition wird unterhalb der Exposition liegen, die bei parenteraler oder oraler Anwendung zur zuverlässigen Ovulationshemmung führen würde. Für orale Anwendungen wird die ovulationshemmende Dosis in der Literatur und in Beispielen in der vorstehenden Tabelle angegeben.

Wenn die ovulationshemmende Dosis für ein gegebenes Gestagen nicht bekannt ist, wird die bei einer parenteralen Arzneiform zu verwendende Freisetzungsrate in einer pharmakokinetischen/pharmakodynamischen Untersuchung ermittelt, wobei die ovariellen, zervikalen und hormonellen Wirkungen verschiedener Dosierungen eines zu verwendenden Gestagens gemessen werden (Ovaraktivität durch transvaginalen Ultraschall, Hormonspiegel im Blut, Insler-Score am Zervixschleim). Als Beispiel für eine nicht sicher ovulationshemmende, aber lokal wirksame Dosis entspricht die systemische Exposition von Levonorgestrel (LNG) nach Freisetzung aus dem IVR einer Exposition von Levonorgestrel nach oraler Gabe in einer Dosierung von mehr als 10 $\mu$g aber weniger als 50 $\mu$g pro Tag.

Dem Fachmann ist bekannt, dass es bei einem IVR auf Polymerbasis kurz nach dem Einführen zu einer erheblich erhöhten Wirkstofffreisetzung kommen kann (der so genannte Bursteffekt). IVRs auf Polymerbasis, die einen solchen Bursteffekt kurz nach dem Einführen zeigen, werden auch als beansprucht angesehen, selbst wenn die Freisetzungsrate während der Dauer des Bursteffekts erhöht ist.

Ein **Aromatasehemmer (AI) in einer täglichen Freisetzungsrate, die keine Stimulation der Ovarien über negative Rückkopplung der Hypophysen-Ovar-Achse induziert** (keine Steigerung der Sekretion von Gonadotropinen, die eine Stimulation von Follikelwachstum induzieren würde) bedeutet die höchste Dosis, die kein zusätzliches Follikelwachstum im Vergleich zum gestagenbehandelten Zyklus induziert, wie durch Bestimmung von Bluthormonspiegeln (follikelstimulierendes Hormon = FSH, Luteinisierungshormon = LH, Östradiol, Progesteron) und transvaginale Ultraschallmessungen untersucht wird.

Wenn sie für einen gegebenen AI nicht bekannt ist, wird die bei einer parenteralen Arzneiform zu verwendende Freisetzungsrate gemäß Beispiel 2 der vorliegenden Anmeldung bestimmt. Bei Anastrozol beträgt die durch die Arzneiform erreichte systemische Exposition im Mittel weniger als die durch 1 mg (oder zwischen 0,1 mg und 0,9 mg) pro Tag/oral hervorgerufene Exposition. Pharmakokinetische Akkumulationsphänomene sind dabei zu berücksichtigen.

Dem Fachmann ist bekannt, dass es bei einem IVR kurz nach dem Einführen zu einer erheblich erhöhten Wirkstofffreisetzung kommen kann (der so genannte Bursteffekt). IVRs auf Polymerbasis, die einen solchen Bursteffekt kurz nach dem Einführen zeigen, werden als beansprucht angesehen, auch wenn die Freisetzungsrate während der Dauer des Bursteffekts erhöht ist.

Die Anwendung in einem IVR stellt eine günstige Formulierung mit geringer Variabilität der Arzneimittel-Serumspiegel bereit, bei der der Metabolismus des Arzneistoffs in der ersten Leberpassage vermieden und die Behandlungscompliance verbessert wird, da es nicht notwendig ist, sich täglich an die Arzneimittelaufnahme zu erinnern. Insbesondere würde das empfängnisverhütende Prinzip der Gestagenpille (POP, "Progestin only pill") in einer Dosierung unterhalb der Ovulationshemmdosis ein genaues Einnahmeschema erfordern, um eine zuverlässige empfängnisverhütende Wirkung zu gewährleisten. In dieser Hinsicht ist die kontinuierliche Verabreichung mittels eines IVR sehr vorteilhaft. Die lokale Anwendung ermöglicht eine geeignete Dosierung, um das gewünschte medizinische Ergebnis zusammen mit einer Verringerung wesentlicher Nebenwirkungen, die mit der systemischen Exposition gegenüber den Wirkstoffen verbunden sind, zu erzielen. Dem Fachmann ist bekannt, dass es bei Anwendung eines IVR (oder anderweitigen Depotformulierungen, insbesondere auch bei polymerbasierten Arzneiformen) zu einer Veränderung (Abfall) der täglichen Freisetzungsrate über den Verabreichungszeitraum kommen kann. Ein IVR welches eine derartige Veränderung zeigt, wird als beansprucht angesehen.

Die IVRs enthalten als Aromataseinhibitor Anastrozol. Bei dem Anastrozol enthaltenden IVR entspricht die erreichte systemische Anastrozolexposition nach Freisetzung aus dem IVR der Exposition von Anastrozol nach oraler Gabe in einer Dosierung von zwischen 0,1 mg und 0,9 mg Anastrozol pro Tag . Dieses IVR enthält als Gestagen Levonorgestrel.

Bei dem IVR entspricht die erreichte systemische Levonorgestrelexposition nach Freisetzung aus dem IVR der Exposition von Levonorgestrel nach oraler Gabe in einer Dosierung von mehr als 10 $\mu$g aber weniger als 50 $\mu$g pro Tag. Beansprucht wird ein gleichzeitig Anastrozol als Aromataseinhibitor und Levonorgestrel als Gestagen enthaltender IVR zur spezifischen Anwendung bei der Behandlung der Endometriose bei dem die erreichte systemische Anastrozolexposition nach Freisetzung aus dem IVR der Exposition von Anastrozol nach oraler Gabe in einer Dosierung von zwischen 0,1 mg und 0,9 mg Anastrozol pro Tag und bei dem die erreichte systemische Levonorgestrelexposition nach Freisetzung aus dem IVR der Exposition von Levonorgestrel nach oraler Gabe in einer Dosierung von mehr als 10 $\mu$g aber weniger als 50 $\mu$g pro Tag entspricht, wobei der Intravaginalring kein Östrogen enthält.

Die Dauer der Langzeitfreisetzung beträgt im Falle des besonders bevorzugten IVR eine Woche bis drei Monate, besonders bevorzugt 4 bis 6 Wochen. Bei den erfindungsgemäßen Arzneiformen kann es aufgrund des Bursteffekts dazu kommen, dass die erfindungsgemäß erwünschten Freisetzungsraten erst ein, zwei oder drei Tage in Ausnahmefällen auch erst eine Woche nach Behandlungsbeginn erreicht werden. Behandlungsbeginn ist dabei der Zeitpunkt der Applikation der Arzneiform.

Alle hier genannten bevorzugten Ausführungsformen dienen der Behandlung der Endometriose. Besonders bevorzugt ist die Behandlung der Endometriose bei gleichzeitiger Kontrazeption. Ebenfalls besonders bevorzugt ist eine Methode zur gleichzeitigen Behandlung der Endometriose und zur Kontrazeption gegebenenfalls unter Verwendung einer der oben genannten bevorzugten Arzneiformen

## AUSFÜHRLICHE BESCHREIBUNG EINER PARENTERALEN ARZNEIFORM

Parenterale Arzneiformen, einschließlich beispielsweise Implantate, intrauterine Device und Intravaginalringe, die eine kontrollierte Freisetzung von Wirkstoffen über ausgedehnte Zeiträume bereitstellen können, werden typischerweise aus biologisch verträglichen Polymeren hergestellt und enthalten ein oder mehrere Arzneimittel, die durch Diffusion durch die Polymermatrix freigesetzt werden. In der Literatur sind mehrere verschiedene Bauarten von Arzneiformen bekannt. Einige Arzneiformen können eine Polymermatrix umfassen, aber keine Membran oder Wand, die die Matrix umhüllt (monolithische Arzneiform), während einige andere Arzneiformen eine Polymermatrix, einen Kern, umfassen, die/der von einer Membran umhüllt ist. Die gleichzeitige Verabreichung von zwei oder mehr therapeutischen Wirkstoffen wird weit verbreitet verwendet, und aus der Literatur sind mehrere verschiedene Bauarten der Arzneiformen bekannt.

Gemäß einer Ausführungsform der Erfindung umfasst die Arzneiform wenigstens eine Kammer, die einen Kern umfasst, oder einen Kern, der von einer Membran umhüllt ist, wobei der Kern und die Membran die gleiche oder unterschiedliche Polymerzusammensetzungen umfassen, wobei wenigstens eine der Kammern Anastrozol umfasst, und gegebenenfalls wenigstens eine Kammer, die die gleiche sein kann wie die Kammer, die Anastrozol umfasst, oder davon verschieden, Levonorgestrel als Gestagen umfasst.

Somit umfasst die Kammer im Wesentlichen eine Polymerzusammensetzung, wobei die Polymerzusammensetzung des Kerns, der Membran oder von beiden einen therapeutischen Wirkstoff oder therapeutische Wirkstoffe umfassen kann. Die Polymerzusammensetzung kann geeignet ausgewählt werden, so dass die Freisetzung des therapeutischen Wirkstoffs durch den Kern, die Membran oder beide reguliert wird.

Gemäß der Ausführungsform, bei der die Arzneiform zwei oder mehr Kammern umfasst, können die Kammern einander benachbart angeordnet sein, nebeneinander, aufeinander, oder zumindest teilweise ineinander, und sie können durch eine Trennmembran oder eine inerte Placebokammer voneinander getrennt sein. Kammern können massiv oder hohl sein.

Die Membran, falls eine vorhanden ist, kann die gesamte Arzneiform bedecken oder nur einen Teil der Arzneiform bedecken, wodurch der Grad der Ausdehnung abhängig von mehreren Faktoren, beispielsweise der Auswahl der Materialien und der Auswahl der Wirkstoffe, variieren kann. Die Membran kann aus mehr als einer Schicht bestehen. Die Dicke der Membran hängt von den verwendeten Materialien und Wirkstoffen sowie von dem gewünschten Freisetzungsprofil ab, im Allgemeinen ist die Dicke aber kleiner als die Dicke des Kernelements.

Die Polymerzusammensetzungen des Kerns, der Membran und der möglichen Trennmembran oder der inerten Placebokammer können gleich oder voneinander verschieden sein und können ein einziges Polymer oder ein Polymergemisch darstellen, oder sie können aus miteinander gemischten Polymeren hergestellt sein.

Im Prinzip kann jedes biologisch abbaubare oder nicht biologisch abbaubare Polymer verwendet werden, solange es biologisch verträglich ist. Beispiele von gewöhnlich verwendeten Polymermaterialien umfassen, sind aber nicht darauf beschränkt, Polysiloxane, Polyurethane, thermoplastische Polyurethane, Ethylen/Vinylacetat-Copolymere (EVA) und Copolymere von Dimethylsiloxanen und Methylvinylsiloxanen, biologisch abbaubare Polymere, beispielsweise Poly(hydroxyalkansäuren), Poly(milchsäuren), Poly(glycolsäuren), Poly(glycolide), Poly(L-lactide), Poly(lactid-co-glycolide) und ein Gemisch von wenigstens zwei davon.

Die strukturelle Integrität des Materials kann durch Zugeben eines teilchenförmigen Materials, wie z. B. Siliciumoxid oder Kieselerde, verstärkt werden. Die Polymerzusammensetzung kann auch zusätzliches Material umfassen, beispielsweise zum Einstellen von hydrophilen oder hydrophoben Eigenschaften, um die gewünschte Freisetzungsrate eines oder mehrerer der Therapeutika zu erzielen, wobei beachtet wird, dass alle Zusatzstoffe biologisch verträglich und für die Patientin unschädlich sein müssen. Der Kern oder die Membran können beispielsweise auch komplexbildende Mittel umfassen, wie z. B. Cyclodextrinderivate, um den Anfangsburst des Stoffes auf das akzeptierte oder gewünschte Niveau einzustellen. Es können auch Hilfsstoffe zugegeben werden, wie z. B. Tenside, Antischaummittel, Stabilisatoren, Solubilisatoren oder Absorptionsverzögerer, oder ein Gemisch von zwei oder mehreren derartiger Stoffe, um dem Körper der Arzneiform gewünschte physikalische Eigenschaften zu verleihen. Außerdem können dem Körper der Arzneiform oder der Membran oder beiden Zusatzstoffe wie Pigmente, Glanzmittel, Mattierungsmittel, Farbmittel, Glimmer oder dergleichen zugegeben werden, um die Arzneiform ein gewünschtes optisches Aussehen zu verleihen.

## HERSTELLUNG EINER PARENTERALEN ARZNEIFORM

Die parenterale Arzneiform gemäß der vorliegenden Erfindung kann gemäß Standardverfahren, die im Fachgebiet bekannt sind, hergestellt werden, und Gestaltung und Größe der Arzneiform können vom Fachmann frei gewählt werden.

Der Polymerzusammensetzung des Kerns oder der Membran kann unter Verwendung verschiedener Verfahren eine ausreichende Menge von wenigstens einem therapeutischen Wirkstoff beigemischt werden, wobei das Verfahren von der Stabilität des Stoffes abhängt. Beispielsweise kann der Stoff homogen mit der Polymermatrix gemischt werden, oder das Polymermaterial und der Stoff können in einem geeigneten Lösungsmittel oder Lösungsmittelgemisch (Dichlormethan, Tetrahydrofuran usw.) gelöst werden, dann kann der größte Teil des Lösungsmittels unter verringertem Druck entfernt werden, um die viskose Lösung kristallisieren zu lassen, gefolgt von weiterem Trocknen und Granulieren der Arzneimittel-Polymer-Zusammensetzung. Der therapeutische Wirkstoff kann auch in ein geschmolzenes Polymer eingemischt werden, insbesondere wenn thermoplastische Elastomere verwendet werden, gefolgt von Abkühlen des Gemisches. Anschließend wird die Arzneimittel-Polymer-Zusammensetzung zu der gewünschten Form verarbeitet, wobei bekannte Verfahren verwendet werden, wie z. B. Formen, Spritzgießen, Rotations/Spritzgießen, Gießen, Extrusion, wie z. B. Coextrusion, Beschichtungsextrusion und/oder Mischextrusion, und andere geeignete Verfahren.

Das Material für die Membran, mit oder ohne therapeutischen Wirkstoff, kann gemäß den vorstehend beschriebenen Verfahren hergestellt werden. Die Membran kann beispielsweise durch Gießen, Sprühen oder Eintauchen, mittels Beschichtungsextrusion oder Coextrusionsverfahren, oder durch mechanisches Strecken oder Dehnen einer vorgefertigten, rohrförmigen Membran durch unter Druck stehendes Gas, beispielsweise Luft, oder durch Quellen in einem geeigneten Lösungsmittel, beispielsweise Propanol, Isopropanol, Cyclohexan, Diglyme und dergleichen, auf die Kerne aufgebracht werden.

Der so erhaltene Polymerstab kann in Stücke mit der gewünschten Länge geschnitten werden, um eine Kammer zu bilden, die einen Kern oder einen von einer Membran umhüllten Kern umfasst. Die Kammer oder zwei oder mehrere miteinander verbundene Kammern können als zu einer im Wesentlichen ringförmigen Arzneiform zusammengesetzt werden. Der Begriff "im Wesentlichen ringförmig" ist so zu verstehen, dass er neben ringförmigen Arzneiformen auch alle anderen im Wesentlichen ringförmigen Strukturen umfasst, die für die vaginale Verabreichung geeignet sind, beispielsweise helikal gewundene Spiralen und Ringsysteme mit einer gewundenen Oberfläche.

Die Enden der Kammern oder der Kombination von Kammern können miteinander verbunden werden, wobei ein Kupplungsmittel verwendet wird, das ein beliebiges Verfahren, ein beliebiger Mechanismus, eine beliebige Vorrichtung oder ein beliebiges Material sein kann, das im Fachgebiet bekannt ist, um Materialien oder Strukturen miteinander zu verbinden. Das Kuppeln kann beispielsweise Verbinden mit Lösungsmittel, Verbinden mit Klebstoff, Wärmeverschmelzen, Verbinden mit Wärme, Druck usw. umfassen. Rohrförmige Kammern können auch unter Verwendung eines Stopfens oder Zapfens miteinander verbunden werden, der aus einem beliebigen inerten, biologisch verträglichen Material hergestellt ist, beispielsweise einem inerten Material, das den Transport von Wirkstoff nicht zulässt. Außerdem können im Wesentlichen ringförmige Arzneiformen durch Anordnen einer Kammer oder einer Kombination von Kammern in einem Formwerkzeug bei erhöhter Temperatur und Einspritzen von geschmolzenem Polyethylen mit hoher Dichte zwischen die Enden hergestellt werden, worauf der hergestellte Ring abgekühlt wird, oder durch Verbinden der Enden miteinander durch Verschweißen.

**Referenzbeispiel 1: Bestimmung der erfindungsgemäßen Gestagendosis durch Untersuchung der Ovulationshemmung**

Bei einer Untersuchung der Ovulationshemmung wird das betrachtete Gestagen in verschiedenen Dosierungen geprüft, wobei die Gestagenwirkung auf die ovarielle Follikelreifung und die Ovulation mithilfe transvaginaler Ultraschalluntersuchungen und Messungen von Bluthormonspiegeln (Östradiol, Progesteron) bestimmt wird. Ferner wird der Zervixschleim gemäß des Insler-Scores auf beabsichtigte Veränderungen der Schleimeigenschaften untersucht, die für Verfahren der Empfängnisverhütung, die nur auf Gestagen beruhen, typisch sind (Insler V et al., Int. J. Gynecol. Obstet .1972, 10(6): 223-228). Die Dosis, die die Ovulation um weniger als 95 % hemmt, vorzugsweise im Bereich von etwa 40-80 %, und einen Insler-Score des Zervixschleims von < 9 erzielt, wird als Gestagendosis bei der vorliegenden Erfindung gewählt werden. Diese Dosis wird für jedes Gestagen spezifisch sein. Dem Fachmann ist bekannt und somit wird erwartet, dass bei diesem Verfahren der Empfängnisverhütung etwas Follikelwachstum stattfindet (beispielsweise ist das Auftreten persistierender Ovarialfollikel eine bekannte Wirkung der Gestagenpille Microlut®; siehe Fachinformation Microlut, datiert Juli 2007, Seite 2 [4.4.2 Warnhinweise; persistierende Ovarialfollikel]). Bei der Identifizierung der Dosis sind pharmakokinetische Akkumulationsphänomene zu berücksichtigen.

**Referenzbeispiel 2: Wirkungen eines Aromatasehemmers auf die Hypophysen-Ovar-Achse und die Follikelentwicklung**

Bei einer weiteren pharmakodynamischen Untersuchung wird die Wirkung des AIs, der über eine parenterale Arzneiform angewendet wird, vorzugsweise über einen IVR, auf die Hypophysen-Ovar-Achse und die Follikelentwicklung durch Bestimmung von Bluthormonspiegeln (follikelstimulierendes Hormon = FSH, Luteinisierungshormon = LH, Östradiol, Progesteron) und transvaginale Ultraschallmessungen allein und/oder in Kombination mit einem Gestagen untersucht. Die niedrigste Exposition mit AI und Gestagen, die zusätzliches Follikelwachstum im Vergleich zu dem unbehandelten oder mit Gestagen behandelten Zyklus induziert, kann als Schwellenwert für die Dosierung des AI in Kombination mit Gestagen verwendet werden. Diese Dosis wird für jeden AI spezifisch sein. In der Literatur wird beschrieben, dass eine ovarielle Stimulation durch einen AI bei Dosierungen von beispielsweise 2.5 mg Letrozol oder 1 mg Anastrozol, oral angewendet, auftreten kann (Mitwally MF & Casper RF, Fertil. Steril. 2001, 75(2): 305-9, Fisher SA et al., Fertil. Steril. August 2002; 78(2): 280-5, Badawy A et al., Fertil. Steril. 2008, 89(5): 1209-1212, Wu HH et al., Gynecol. Endocrinol. 2007, 23(2): 76-81). Die angestrebte mittlere tägliche Exposition von beispielsweise Anastrozol, die über die bevorzugte parenterale Arzneiform abgegeben wird, die bei der vorliegenden Erfindung ein IVR oder IUD ist, wird unter 1 mg liegen (oder zwischen 0,1 mg und 0,9 mg). Bei Letrozol wird sie unter 2,5 mg liegen (oder zwischen 0,1 mg und 2,4 mg).

Mittels der oben beschriebenen pharmakodynamischen Untersuchung am Menschen wird die größtmögliche Menge an AI in Kombination mit der vorstehend beschriebenen Gestagendosis bestimmt, die zu keiner zusätzlichen Stimulation des Follikelwachstums im Vergleich zu dem wie vorstehend bestimmten Gestagen allein führt. Die Gestagenwirkung auf den Zervixschleim muss bei der Kombination mit AIs erhalten bleiben.

Der experimentelle Aufbau ist für alle parenteralen Anwendungen gültig. Für einen IVR würden die vorstehend beschriebenen Experimente für die einzelnen Komponenten und für die Kombination mit IVRs durchgeführt werden.

**Beispiel 3: Herstellung der Intravaginalringe für die in-vivo-Studie**

Für eine *in-vivo*-Studie mit Cynomolgus Affen wurden anastrozolfreisetzende, an den Cynomolgus-Affen größenangepasste Intravaginalringe angefertigt. Die Ringe hatten einen Außendurchmesser von 14 mm und einen Querschnitt von 2,3 mm.

Die Ringe enthielten einen Kern aus Anastrozol und Elastomer, der mit einer die Freisetzung steuernden Membran überzogen war. Die vorgesehenen Arzneimitteldosierungen wurden durch eine geeignete Auswahl der Materialien für den Kern und die Membran und durch Einstellen der Arzneimittelkonzentration und der Oberfläche des anastrozolhaltigen Kerns in Kombination mit der Membrandicke erzielt. Durch eine geeignete Auswahl dieser Parameter lässt sich die Freisetzung von Anastrozol über mehr als 30 Tage steuern.

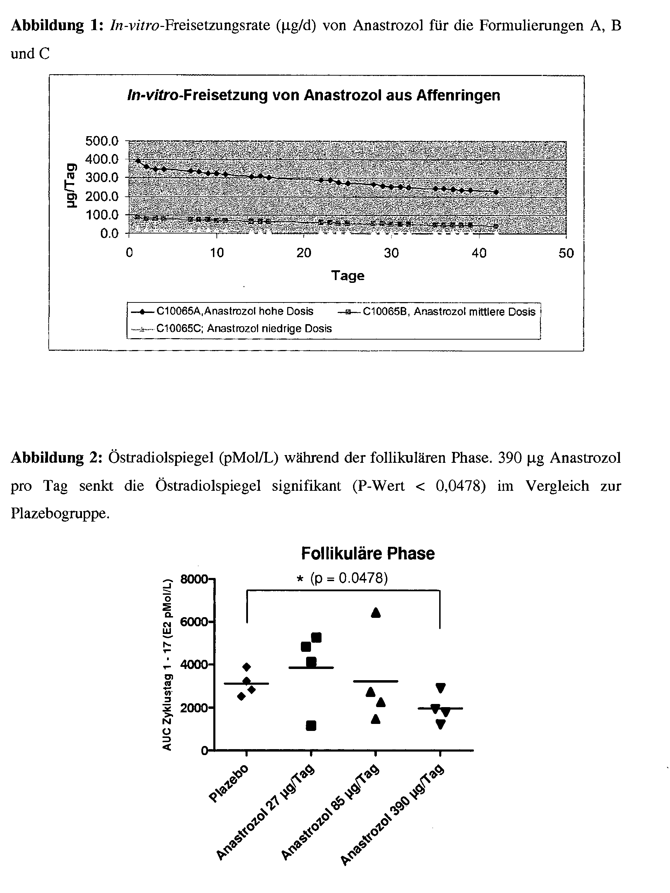

Es wurden drei Formulierungen (A, B, C; in Abbildung 1 als hohe, mittlere und geringe Dosis bezeichnet) von anastrozolfreisetzenden Ringen hergestellt, die jeweils mindestens 30 Tage lang Anastrozol freisetzten. Die Ausgangsdosierungen von Anastrozol betrugen 390 μg/Tag (A), 85 μg/Tag (B) bzw. 27 μg/Tag (C). Placeboringe wurden ebenfalls hergestellt.

**a) Herstellung der anastrozolfreisetzenden Ringe**

Kern

Es wurden zwei Kernzusammensetzungen zubereitet, wobei die eine Anastrozol in einer Matrix aus Siliconelastomer (Polydimethylsiloxan) und die andere nur das Siliconelastomer (Polydimethylsiloxan) enthielt. Der anastrozolhaltige Kern wurde hergestellt, indem man in einem Mischer (mikronisiertes) Anastrozol mit dem Siliconelastomer mischt. Der Anastrozolgehalt der Mischung betrug 35 Gew.-%. Die Mischung wurde in einer Form zu einem elastischen Stäbchen mit einer Dicke von 2 mm geformt und gehärtet (dies hätte auch durch Extrudieren durch eine Düse erfolgen können). Der Siliconelastomerkern wurde zu einem elastischen Stäbchen mit einer Dicke von 2 mm extrudiert (dies hätte auch in einer Form erfolgen können).

Membran

Der die Arzneimittelfreisetzung steuernde Membranschlauch wurde durch Schlauchextrusion aus Siliconelastomer (Polydimethylsiloxan) hergestellt. Die Wanddicke des Schlauchs (die Membrandicke) betrug etwa 1,5 mm.

Zusammenbau des Rings

Der Anastrozolkern wurde in drei Längen geschnitten: 38 mm (A), 6 mm (B) und 1,5 mm (C). Der Siliconelastomerkern wurde in zwei Längen geschnitten, so dass man eine Gesamtkernlänge von 38 mm erzielte. Der Membranschlauch wurde zu einer Länge von 38 mm geschnitten und in Cyclohexan aufgequollen.

Der Ring wurde zusammengesetzt, indem man das/die Kernsegment(e) in den aufgequollenen Membranschlauch gab. Der Schlauch wurde durch Überlappen zu einem Ring geformt. Nach dem Abdampfen des Lösungsmittels schrumpfte der Schlauch und drückte die Teile eng zusammen.

**Anastrozolfreisetzung**

Methode

Die Freisetzung von Anastrozol aus den Ringen wurde *in vitro* bei 37°C in einer 1,0%igen wässrigen Lösung von 2-HP-β-CD (2-Hydroxypropyl-beta-cyclodextrin) in einem Schüttelbad (100 U/min) analysiert. Die Lösungen wurden bis auf die Wochenenden täglich gewechselt. Die Probenlösungen wurden durch HPLC analysiert, wobei eine Inertsil ODS-3, 150 x 4 mm 5 μm-Säule und Methanol/Wasser (1/1) als Laufmittel bei einer Fließgeschwindigkeit von 1,0 ml/min verwendet wurden. Die Detektionswellenlänge für Anastrozol war 215 nm. Es wurden drei Ringe parallel getestet.

*In-vitro*-Freisetzungsrate

Die Ringe wurden bis zu 40 Tage lang *in vitro* getestet. Die *in-vitro*-Freisetzungsrate war kontinuierlich und gesteuert, zeigte jedoch beim Testen eine Abnahme vom Ausgangswert von insgesamt etwa 30% nach 30 Tagen. Die Ausgangsfreisetzungsraten betrugen 390 μg/Tag (A), 85 μg/Tag (B) bzw. 27 μg/Tag (C), und die mittlere Freisetzung während der 30 Tage betrug 305 μg/Tag (A), 64 μg/Tag (B) und 16 μg/Tag (C).
Die *in-vitro*-Freisetzungsrate von Anastrozol ist in Abbildung 1 beschrieben.

*Ex-vivo*-Untersuchung der Affenringe

Die verwendeten Ringe (5) der jeweiligen Dosen (A, B und C) wurden geborgen und auf den Anastrozolrestgehalt analysiert. Der Anastrozolgehalt wurde bestimmt, indem man den Ring mit (THF) extrahierte, gefolgt von HPLC-Analysen.
Ein Schätzwert für die Freisetzung von Anastrozol *in vivo* wurde erhalten, indem man die Abnahme der Menge an Anastrozol im Ring während des Gebrauchs berechnete, z.B. den ursprünglichen Gehalt minus den *ex-vivo*-Restgehalt, und dies durch die Anzahl von Tagen, an denen sich der Ring in Gebrauch befand, dividierte (verschieden). In Tabelle 1 wird der durchschnittliche (5 Ringe) *ex-vivo*-Anastrozolgehalt pro Dosis und der Anastrozolgehalt in den Vergleichsringen (nicht verwendeten Ringen) zusammen mit der berechneten durchschnittlichen Anastrozolfreisetzungsrate pro Tag aufgeführt.

**Tabelle 1.** Schätzwert für die Anastrozolfreisetzung pro Tag *in vivo* für die Dosen A, B und C, berechnet aus der durchschnittlichen in-vivo-Testdauer und den durchschnittlichen Assayergebnissen für die *ex-vivo*-Ringe und die nicht verwendeten Vergleichsringe

| Dosis | Durchschnittlicher Assaywert für die ex-vivo-Ringe (mg) | Durchschnittlicher Assaywert für die Vergleichsringe (mg) | Durchschnittliche Freisetzungsrate pro Tag (μg/Tag) |
|---|---|---|---|
| A | 32,8 | 41,1 | 277 |
| B | 4,9 | 6,5 | 54 |
| C | 1,1 | 1,5 | 15 |

**Beispiel 4: Nachweis der Machbarkeit im Cynomolgen**

Der Cynomolgous Affe eignet sich als Modell-Tier zur Untersuchung von humanen endokrinen Aspekten aufgrund seines mit dem Menschen vergleichbaren reproduktiven Systems (Weinbauer, N., Niehaus, Srivastav, Fuch, Esch, and J. Mark Cline (2008). "Physiology and Endocrinology of the Ovarian Cycle in Macaques." Toxicologic Pathology 36(7): 7S-23S). Dieses umfasst u.a. die Zykluslänge, Hormonrezeptoren, Morphologie, endokrines System und die Regulation der Hypophysen-Ovar-Achse (Borghi, M. R., R. Niesvisky, et al. (1983). "Administration of agonistic and antagonistic analogues of LH-RH induce anovulation in Macaca fasicularis." Contraception 27(6): 619-626. Satoru Oneda, T. I., Katsumi Hamana (1996). "Ovarian Response to Exogenous Gonadotropins in Infant Cynomolgus Monkeys " International Journal of Toxicology, 15(3): 194-204). Der pharmakodynamische und pharmakokinetische Effekt von intravaginal verabreichten Dosierungen des Aromatasehemmers Anastrozol wurde durch Einsetzen eines Vaginalrings (IVR) mit drei verschiedenen Freisetzungsraten über die Dauer eines Menstrual-Zykluses untersucht. Durch Bestimmung der Hormone Östradiol, FSH, Progesteron (die dafür nötigen Blutabnahmen erfolgten über den gesamten Versuchszeitraum, an Tag 1 vier Entnahmen [0h, 1h, 3h, 6h nach Einsetzen des IVRs], jeweils 1 Entnahme an Tag 2 und 3, ab diesem Zeitpunkt folgten weitere Entnahmen an jedem 3. Tag) und durch Ultraschalluntersuchungen des Ovars (2 x pro Woche) wurde u.a. der Einfluss auf die Hypophysen-Ovar-Achse untersucht. Die Hormonbestimmungen wurden nach Vorschrift der Anbieter durchgeführt (Östradiol [Siemens/DPC], Progesteron [Beckmann-Culter/DSL], FSH [SHG]).

Fünf Tiere pro Gruppe wurde ein IVR mit einer initialen *in vitro* Freisetzung von 0 $\mu$g/Tag (Plazebo, ohne Anastrozol), 390 $\mu$g/Tag, 85 $\mu$g/Tag, und 27 $\mu$g/Tag ein bis drei Tage nach dem letzten Tag der Regelblutung eingesetzt. Tiere mit unregelmäßigen Zyklus wurden aus dem Versuch ausgeschlossen.

Eine Abnahme der Östradiolspiegel über den gesamten Zyklus mit einem signifikanten Abfall während der - für die östrogenabhängige Proliferation des Endometriums und endometriotischer Läsionen entscheidenden - follikulären Phase wurde in der Gruppe mit einer initialen Freisetzung von 390 $\mu$g/Tag beobachtet (Tabelle 2, Zeile 5 und Abbildung 2). Wie in Zeilen 1, 2 und 3 der Tabelle 2 dargestellt, bleibt die Gegenregulation durch die Hypophysen-Ovar-Achse in den verwendeten Dosierungen aus (kein Unterschied zur Plazebokontrolle). Vergleichbare FSH-Spiegel zwischen den Gruppen zeigen, dass die verwendeten Dosierungen keine Stimulierung der Hypophysen-Ovar-Achse bewirken.

Übereinstimmend mit dieser Beobachtung wurde keine Bildung ovarieller Zysten (vergl. Zeile 7, Tabelle 2). Dieses Experiment zeigt, dass es im Tiermodell möglich ist mit einem Aromataseinhibitor (beispielsweise Anastrozol) die endogenen Estrogenspiegel abzusenken ohne eine Gegenregulierung auzulösen.

Die folgenden Tabellen enthalten eine Zusammenfassung der *in vivo* und *in vitro* Freisetzungsraten [Tabelle 1] von Anastrozol aus dem IVR, die Östradiol (E2), Progesteron und FSH Spiegel unter verschiedenen Dosierungen von Anastrozol, sowie Angaben über die Bildung von ovariellen Zysten während des Zyklusses (Tag 1-26) [Tabelle 2].

**Tabelle 1:** Zusammenfassung der *in vivo* und *in vitro* Freisetzungsraten

| | Anastrozol |
|---|---|
| Initiale (Tag1) *in vitro* Freisetzung ($\mu$g/Tag) | |
| (A) | **390** |
| (B) | **85** |
| (C) | **27** |
| Durchschnittliche (30 Tage) *in vitro* Freisetzung ($\mu$g/Tag) | |
| (A) | **305** |
| (B) | **64** |
| (C) | **16** |
| Durchschnittliche (30 Tage) *in vivo* Serumkonzentration ($\mu$g/L) | |
| (A) | **5.9** |
| (B) | **1.4** |
| (C) | **0.3** |
| Durchschnittliche (30 Tage) *in vivo* Freisetzung ($\mu$g/Tag) (PK basierend) | |
| (A) | **278** |
| (B) | **66** |
| (C) | **16** |
| Basierend auf der ex-vivo IVR Analyse | |
| (A) | **277** |
| (B) | **54** |
| (C) | **15** |
| **Plasmaproteinbindung [freie Fraktion,** fu] | |
| Cynomolge | **34**% |
| Human | **52**% |
| **CL**$_{pl}$ [L/h/kg] | |
| Cynomolge | **0.58** |
| Human (CL/F) | **0.02** |
| Errechnete konstante *in vivo* IVR Freisetzungsrate im Menschen (zum Erhalt von Plasmaspiegeln, die denen der effektiven Dosis im Cynomolgen entsprechen) | $\approx$ **250 $\mu$g/Tag/60kg Patient** |
| Errechnete konstante *in vitro* IVR Freisetzungsrate (in Puffer) (um im Menschen Plasmaspiegel zu erhalten, die denen der effektiven Dosis im Cynomolgen entsprechen) | $\approx$ **270 $\mu$g/Tag/60kg Patient** |

(fortgesetzt)

| | Anastrozol |
|---|---|
| Errechnete initiale *in vitro IVR* Freisetzungsrate (in Puffer) mit einer abnehmenden Freisetzungsrate (32% in 4 Wochen) (um im Menschen Plasmaspiegel zu erhalten, die denen der effektiven Dosis im Cynomolgen entsprechen) (humane Dosis) | ≈ 350 μg/Tag/60kg Patient |

Tabelle 2: Östradiol (E2), Progesteron und FSH Spiegel und die Bildung von ovariellen Zysten während des Zykluses (Tagl-26).

| | | Plazebo | Anastrozol 27 μg/Tg (Initiale *in vitro* Freisetzung), | Anastrozol 85 μg/Tag (Initiale *in vitro* Freisetzung) | Anastrozol 390 μg/Tag (Initiale *in vitro* Freisetzung) | P Wert vs Plazebo |
|---|---|---|---|---|---|---|
| 1 | FSH (μg/L) Ø Spiegel/Tag ohne präovulatorisches Maximum | 4,85 +/- 2,70 | 5,52 +/- 3,07 | 4,90 +/- 2,58 | 4,83 +/- 2,91 | nicht signifikant |
| 2 | Progesteron (nMol/L) Ø Spiegel/Tag follikuläre Phase | 5,65 +/- 5,99 | 5,57 +/- 5,11 | 6,58 +/- 3,91 | 4,58 +/- 2,64 | nicht signifikant |
| 3 | Progesteron (nMol/1) Ø Spiegel/Tag Lutealphase | 51,61 +/- 37,54 | 91,92 +/- 52,78 | 60,02 +/- 22,65 | 92,88 +/- 55,50 | nicht signifikant |
| 4 | E2 pMol/L AUC (Zyklustag 1-26) | 3768 +/- 684,9 | 4862 +/- 1986 | 4126 +/- 2063 | 2784 +/- 999,8 | nicht signifikant |
| 5 | E2 pmol/L /Tag AUC (follilulär, Zyklustag 1-17) | 3137 +/- 295,5 | 3854 +/- 927,5 | 3235 +/- 1101 | 1978 +/- 350,6 | P< 0,0478 (Anastrozol 390 μg/Tag vs. Plazebo) |
| 6 | E2 pMol/L AUC (luteal, Zyklustag 17-26) | 404 +/- 211,9 | 403,2 +/- 169,7 | 605,1 +/- 264,1 | 342,9 +/- 135,2 | nicht signifikant |
| 7 | Ovarielle Zysten (Ultraschall) | keine | keine | keine | keine | n.a. |

Abbildung 2 zeigt die Östradiolspiegel (pMol/L) während der follikulären Phase. 390 μg Anastrozol pro Tag senkt die Östradiolspiegel signifikant (P-Wert < 0,0478) im Vergleich zur Plazebogruppe.

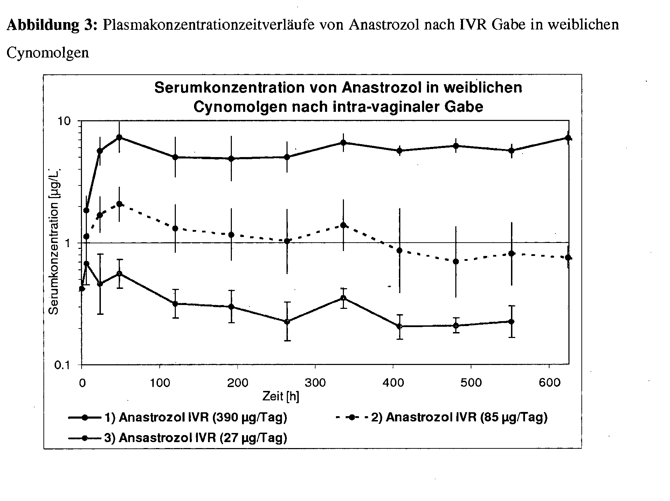

Die quantitative Konzentrationsbestimmung von Anastrozol in Plasmaproben erfolgte nach Flüssig-Flüssigextraktion mit Flüssigkeitschromatografie gekoppelt an Tandemmassenspektrometrie (LC/ESI-MS/MS). Die Analysen wurden an einer Agilent 1200 und einem AB Sciex Triple Quad 5500 im positiven Ionisierungsmodus durchgeführt. Hierzu wurden von jeder Plasmaprobe zunächst 100 μL entnommen, mit 300 μL einer wässrigen Lösung versetzt, welche eine beliebige, nicht strukturverwandte Verbindung als internen Standard enthält, und mit 1.3 mL Methyl-tertiär-butylether auf einer Perkin Elmer Mass Prep Station extrahiert. Nach der Phasentrennung wurde die organische Phase abgeblasen und der Rückstand mit 30 μL LC-Laufmittel (50% Methanol/50% Wasser, v/v) aufgenommen. Daraus wurden 5 μL in das LC/MS/MS injiziert, der m/z-Übergang 294 ([M+H]+) → 225 aufgenommen und das Signal mit der AB Sciex-Software Analyst 1.5 integriert. Aus den resultierenden Flächen wurden die Konzentrationen der Plasmaproben anhand einer in der gleichen Sequenz vorhandenen Kalibrierkurve (0, 0.0500 bis 1000 nM in Plasma, n=2) bestimmt. Die untere Bestimmungsgrenze dieser Methode betrug circa 1,2 μg/L (quadratische Kalibrierkurve, Wichtung 1/x). Die Plasmakonzentrationszeitverläufe von Anastrozol finden sich in Abbildung 3. Die Bestimmung der Plasmaproteinbindung (freie Fraktion [fu]) von Anastrozol in menschlichem und Cynomolgen Plasma erfolgte mittels Gleichgewichtsdialyse (vgl. Banker, M.

J. Banker, et.al. (2003). "Development and Validation of a 96-Well Equilibrium Dialysis Apparatus for Measuring Plasma Protein Binding" J. Pharm. Sci. 92 (5): 967-974) über sieben Stunden bei 37°C, in einer 96-Loch basierten Mikrodialyseapparatur Apparatur (HT-Dialysis LLC) mit einer Dialysemembran aus regenerierter Cellulose (MWCO 3.5K) und nachfolgender Messung der Dialysate mittels LC/ESI-MS/MS. Die Berechung der freien Fraktion (fu) ergab im Menschen 34% und im Cynomolgen 52%.

Abbildung 3 zeigt die Plasmakonzentrationzeitverläufe von Anastrozol nach IVR Gabe in weiblichen Cynomolgen

Die mittlere Plasmakonzentration (Css) von Anastrozol wurde als Mittelwert aller gemessenen Konzentrationen je einer Dosisgruppe vom Tag nach Einsetzen des IVR bis zum Ende des Versuchs berechnet.

Zur Berechnung der *in vivo* Freisetzungsrate von Anastrozol aus dem Vaginalring wurde in einem separaten Versuch die *in vivo* Plasmaclearance (CL) in weiblichen Cynomolgen bestimmt. Dafür wurden weiblichen Cynomolgen Anastrozol mit einer Dosis von jeweils 0.2 mg/kg in 50% PEG400 intravenös verabreicht, zu verschiedenen Zeiten Blutproben genommen und die Plasmakonzentration mittels LC/ESI-MS/MS bestimmt. Die daraus berechnete Plasmaclearance (CL) betrug für Anastrozol 0.58 L/h/kg.

Die mittleren *in vivo* Freisetzungsraten (Rin) aus dem IVR wurden nachfolgend entsprechend der Gleichung: Rin = Css * CL berechnet (s. Tabelle X). Es zeigte sich, dass die so berechneten mittleren Freisetzungsraten gut zu den *in vitro* Freisetzungsraten in Puffer passten (*in vitro*/*in vivo* Korrekturfaktor 1.1). Weiterhin stimmten sie gut überein mit der mittleren *in vivo* Freisetzungsrate, berechnet aus dem ex vivo Restgehalt der getragenen Ringe am Ende der Studie.

Anschließend wurde die *in vitro* IVR Freisetzungsrate eines IVR für die humane Anwendung abgeschätzt, die notwendig ist, um Serumspiegel zu erreichen, die im Affen zu einer Östradiolabsenkung geführt haben. Im Cynomolgen wurde dies in der höchsten Dosisgruppe bei einer mittleren Serumkonzentration (Css) von 5.9 µg/L erreicht. Die entsprechende effektive Serumkonzentration im Menschen wird unter Berücksichtigung der speziespezifischen Plasmaproteinbindung entsprechend nachfolgender Gleichung (1) auf 9 µg/L abgeschätzt.

$$\text{Gleichung 1:} \qquad Css_{human} = Css_{monkey} \cdot \frac{fu_{monkey}}{fu_{human}}$$

Die mittlere *in vivo* Freisetzungsrate aus dem IVR, die zur Erreichung einer Plasmakonzentration von 9 µg/L im Menschen notwendig ist wird entsprechend Gleichung 2 berechnet. Dafür wird die Plasmaclearance von Anastrozol im Menschen benötigt. Diese ist nur nach oraler Gabe (CL/F) bekannt (Clin. Pharmacol. and Biopharmac. Review, NDA 020541 (28.09.1995)) und konnte für die Berechnung als CL verwendet werden, da die orale Bioverfügbarkeit (F) näherungsweise 1 beträgt.

$$\text{Gleichung 2:} \qquad Rin_{human} = Css_{human} \cdot CL_{human}$$

Es ergab sich eine humane *in vivo* Freisetzungsrate von 246 µg/d, die konstant erfolgen muß, um im Menschen Spiegel zu erreichen, die im Affen eine Östradiolabsenkung erreichten. Unter Annahme einer vergleichbaren Permeation von Anastrozol in der Vagina von Affe und Mensch, ergibt sich mit dem aus dem Affenversuch berechneten *in vitro*/*in vivo* Korrekturfaktor von 1.1 eine für den Menschen erforderliche konstante *in vitro* Freisetzungsrate in Puffer von Anastrozol von 270 µg/d. Falls es beim IVR in Menschen einen vergleichbaren Abfall der Freisetzungsrate über die Zeit gibt wie beim Affen, müsste die entsprechende initiale *in vitro* Freisetzungsrate höher sein, sie wurden mit ca. 350 µg pro Tag berechnet (Tabelle 1).

**Abbildungsverzeichnis**

Abbildung 1: *In-vitro*-Freisetzungsrate (µg/d) von Anastrozol für die Formulierungen A (hohe Dosis = 390µg/Tag), B (mittlere Dosis = 85 µg/Tag) und C (geringe Dosis = 27 µg/Tag)

Abbildung 2: Östradiolspiegel (pMol/L) während der follikulären Phase. 390 µg Anastrozol pro Tag senkt die Östradiolspiegel signifikant (P-Wert < 0,0478) im Vergleich zur Plazebogruppe.

Abbildung 3: Plasmakonzentrationzeitverläufe von Anastrozol nach IVR Gabe in weiblichen Cynomolgen

**Patentansprüche**

1. Intravaginalring enthaltend Anastrozol und Levonorgestrel zur spezifischen Anwendung bei der Behandlung der Endometriose, bei dem die erreichte systemische Anastrozolexposition nach Freisetzung aus dem IVR der Exposition von Anastrozol nach oraler Gabe in einer Dosierung von zwischen 0,1 mg und 0,9 mg Anastrozol pro Tag, und bei dem die erreichte systemische Levonorgestrelexposition nach Freisetzung aus dem IVR der Exposition von Levonorgestrel nach oraler Gabe in einer Dosierung von mehr als 10 $\mu$g aber weniger als 50 $\mu$g pro Tag entspricht, wobei der Intravaginalring kein Östrogen enthält.

2. Intravaginalring zur spezifischen Anwendung bei der Behandlung der Endometriose gemäß Anspruch 1 bei der die dort beanspruchten erwünschten Freisetzungsraten aufgrund des Burst-Effekts erst ein, zwei oder drei Tage nach Behandlungsbeginn erreicht werden.

3. Intravaginalring zur spezifischen Anwendung bei der Behandlung der Endometriose nach einem der vorgenannten Ansprüche, wobei die Behandlungsdauer 1 Woche bis 3 Monate beträgt.

4. Intravaginalring zur spezifischen Anwendung bei der Behandlung der Endometriose gemäß Anspruch 3, wobei die Behandlungsdauer 4 bis 6 Wochen beträgt.

**Claims**

1. Intravaginal ring containing anastrozole and levonorgestrel for specific use in the treatment of endometriosis, in which the systemic anastrozole exposure achieved after release from the IVR corresponds to the anastrozole exposure after oral administration in a dosage of between 0.1 mg and 0.9 mg of anastrozole per day, and in which the systemic levonorgestrel exposure achieved after release from the IVR corresponds to the levonorgestrel exposure after oral administration in a dosage of more than 10 ug, but less than 50 $\mu$g, per day, the intravaginal ring containing no estrogen.

2. Intravaginal ring for specific use in the treatment of endometriosis according to Claim 1 in which the desired release rates claimed therein are achieved only one, two or three days after the start of treatment owing to the burst effect.

3. Intravaginal ring for specific use in the treatment of endometriosis according to either of the abovementioned claims in which the treatment period lasts from 1 week to 3 months.

4. Intravaginal ring for specific use in the treatment of endometriosis according to Claim 3 in which the treatment period lasts from 4 to 6 weeks.

**Revendications**

1. Anneau intravaginal contenant de l'anastrozole et du lévonorgestrel destiné à une utilisation spécifique dans le traitement de l'endométriose, dans lequel l'exposition systémique à l'anastrozole atteinte après libération à partir de l'anneau intravaginal correspond à l'exposition à l'anastrozole après prise orale en un dosage compris entre 0,1 mg et 0,9 mg d'anastrozole par jour, et dans lequel l'exposition systémique au lévonorgestrel atteinte après libération à partir de l'anneau intravaginal correspond à l'exposition au lévonorgestrel après prise orale en un dosage de plus de 10 $\mu$g mais de moins de 50 $\mu$g par jour, dans lequel l'anneau intravaginal ne contient aucun oestrogène.

2. Anneau intravaginal destiné à une utilisation spécifique dans le traitement de l'endométriose selon la revendication 1, dans lequel les vitesses de libération désirées revendiquées ici sont atteintes sur la base d'un effet d'éclatement seulement un, deux ou trois jours après le début du traitement.

3. Anneau intravaginal destiné à une utilisation spécifique dans le traitement de l'endométriose selon l'une des revendications précédentes, dans lequel la durée de traitement vaut de 1 semaine à 3 mois.

4. Anneau intravaginal destiné à une utilisation spécifique dans le traitement de l'endométriose selon la revendication 3, dans lequel la durée de traitement vaut de 4 à 6 semaines.

**Abbildung 1:** *In-vitro*-Freisetzungsrate (µg/d) von Anastrozol für die Formulierungen A, B und C

**Abbildung 2:** Östradiolspiegel (pMol/L) während der follikulären Phase. 390 µg Anastrozol pro Tag senkt die Östradiolspiegel signifikant (P-Wert < 0,0478) im Vergleich zur Plazebogruppe.

**Abbildung 3:** Plasmakonzentrationzeitverläufe von Anastrozol nach IVR Gabe in weiblichen Cynomolgen

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 0315872 A
- WO 0469260 A, Amsterdam LL
- US 20050101579 A, Shippen
- WO 0317973 A
- US 20110033519 A1

**In der Beschreibung aufgeführte Nicht-Patentliteratur**

- **URABE M et al.** *Acta Endocrinol.,* 1989, vol. 121 (2), 259-64
- **NOBLE LS et al.** *J. Clin. Endocrinol. Metab.,* 1996, vol. 81 (1), 174-9
- **NOBLE LS et al.** *J. Clin. Endocrinol. Metab.,* 1997, vol. 82 (2), 600-6
- **GEISLER J et al.** *J. Clin. Oncol.,* 2002, vol. 20 (3), 751-757
- **AILAWADI RK et al.** *Fertility & Sterility,* 2004, vol. 81 (2), 290-296
- **AMSTERDAM LL et al.** *Fertility & Sterility,* 2005, vol. 84 (2), 300-304
- **AILAWADI RK et al.** *AI + NETA,* 2004
- *Fertility and sterility,* 2005, vol. 84, ISSN 0015-0282
- **INSLER V et al.** *Int. J. Gynecol. Obstet.,* 1972, vol. 10, 223-228
- **NEUMANN F et al.** *Reproduktionsmedizin,* 1998, vol. 14, 257-264
- **TAUBERT H D ; KUHL, H.** Kontrazeption mit Hormonen. 1995
- **TAUBERT H D ; KÜHL, H.** Kontrazeption mit Hormonen. 1995
- **INSLER V et al.** *Int. J. Gynecol. Obstet .,* 1972, vol. 10 (6), 223-228
- Auftreten persistierender Ovarialfollikel eine bekannte Wirkung der Gestagenpille Microlut. *Fachinformation Microlut,* Juli 2007, 2
- **MITWALLY MF ; CASPER RF.** *Fertil. Steril.,* 2001, vol. 75 (2), 305-9
- **FISHER SA et al.** *Fertil. Steril.,* August 2002, vol. 78 (2), 280-5
- **BADAWY A et al.** *Fertil. Steril.,* 2008, vol. 89 (5), 1209-1212
- **WU HH et al.** *Gynecol. Endocrinol.,* 2007, vol. 23 (2), 76-81
- **WEINBAUER, N. ; NIEHAUS, SRIVASTAV ; FUCH, ESCH ; J. MARK CLINE.** Physiology and Endocrinology of the Ovarian Cycle in Macaques. *Toxicologic Pathology,* 2008, vol. 36 (7), 7S-23S
- **BORGHI, M. R. ; R. NIESVISKY et al.** Administration of agonistic and antagonistic analogues of LH-RH induce anovulation in Macaca fasicularis. *Contraception,* 1983, vol. 27 (6), 619-626
- **SATORU ONEDA, T. I. ; KATSUMI HAMANA.** Ovarian Response to Exogenous Gonadotropins in Infant Cynomolgus Monkeys. *International Journal of Toxicology,* 1996, vol. 15 (3), 194-204
- **BANKER, M. J. BANKER.** Development and Validation of a 96-Well Equilibrium Dialysis Apparatus for Measuring Plasma Protein Binding. *J. Pharm. Sci.,* 2003, vol. 92 (5), 967-974
- *Clin. Pharmacol. and Biopharmac. Review, NDA 020541,* 28. September 1995